# EUROPEAN PATENT SPECIFICATION

(11) **EP 1 296 703 B1**
(45) Date of publication and mention of the grant of the patent: **09.02.2005**
(21) Application number: 01946039.3
(22) Date of filing: 01.06.2001
(51) Int. Cl.: A61P 31/10, A61K 31/65

(54) **NON-ANTIBACTERIAL TETRACYCLINE AS ANTI-FUNGAL AGENTS**
NICHT-ANTIBAKTERIELLE TETRAZYKLINE ALS ANTI-PILZ MEDIKAMENTE
TETRACYCLINE NON ANTIBACTERIENNE EN TANT QU'AGENT ANTIFONGIQUE

(30) Priority: 02.06.2000 US 209097 P
(43) Date of publication of application: 02.04.2003
(73) Proprietor: Liu, Yu, Stony Brook, NY 11790 (US); Golub, Lorne M., Smithdown, NY 11787 (US); Ryan, Maria, Huntington, NY 11743 (US); Lee, Hsi-Ming J., Setauket, NY 11733 (US); Simon, Sanford, Stony Brook, NY 11790 (US)
(72) Inventor: Liu, Yu, Stony Brook, NY 11790 (US); Golub, Lorne M., Smithdown, NY 11787 (US); Ryan, Maria, Huntington, NY 11743 (US); Lee, Hsi-Ming J., Setauket, NY 11733 (US); Simon, Sanford, Stony Brook, NY 11790 (US)
(74) Representative: Prins, Adrianus Willem, Mr. Ir.
(86) International application number: PCT/US2001/017750
(87) International publication number: WO 2001/093891

(56) References cited:
- US-A- 5 223 248
- US-A- 5 770 588
- US-A- 5 827 840
- US-A- 5 886 175
- DATABASE CA [Online] CHEMICAL ABSTRACTS SERVICE, COLUMBUS, OHIO, US; PRUZANSKI, WALDEMAR ET AL: "Chemically modified non-antimicrobial tetracyclines inhibit activity of phospholipases A2" retrieved from STN Database accession no. 130:20186 XP002246102 & JOURNAL OF RHEUMATOLOGY (1998), 25(9), 1807-1812 ,
- DATABASE BIOSIS [Online] BIOSCIENCES INFORMATION SERVICE, PHILADELPHIA, PA, US; May 2002 (2002-05) LIU YU ET AL: "A chemically modified tetracycline (CMT-3) is a new antifungal agent." Database accession no. PREV200200273907 XP002246103 & ANTIMICROBIAL AGENTS AND CHEMOTHERAPY, vol. 46, no. 5, May 2002 (2002-05), pages 1447-1454, May, 2002 ISSN: 0066-4804

## Description

### BACKGROUND OF INVENTION

The invention relates to the inhibition of the growth of fungus. More specifically, the method relates to the use of non-antibacterial tetracycline compounds for the inhibition of fungal growth.

Certain tetracycline compounds, including tetracycline itself, as well as sporocycline, etc., are broad spectrum antibiotics, having utility against a wide variety of bacteria. The parent compound, tetracycline, has the following general structure:

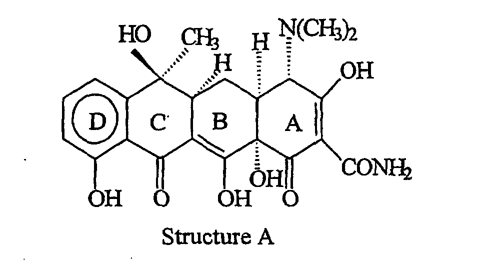

The numbering system for the multiple ring nucleus is as follows:

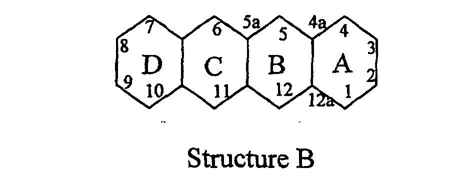

Tetracycline, as well as the 5-OH (terramycin) and 7-Cl (aureomycin) derivatives, exist in nature, and are all well known antibiotics. Semisynthetic derivatives such as 7-dimethylamino-tetracycline (minocycline) and 6α-deoxy-5-hydroxy-tetracycline (doxycycline) are also known antibiotics. Natural tetracyclines may be modified without losing their antibiotic properties, although certain elements of the structure must be retained to do so.

A class of compounds has been defined that are structurally related to the antibiotic tetracyclines, but which have had their antibiotic activity substantially or completely expunged by chemical modification. Modifications to the basic tetracycline structure have been reviewed by Mitscher (1978). According to Mitscher, modification at positions 5-9 of the tetracycline ring system can be made without causing the complete loss of antibiotic properties.

However, changes to the basic structure of the ring system, or replacement of substituents at positions 1-4 or 10-12, generally lead to synthetic tetracyclines with substantially less, or essentially no, antibacterial activity. For example, 4-de(dimethylamino)tetracycline is commonly considered to be a non-antibacterial tetracycline. These compounds, known as chemically-modified tetracyclines (CMTs), have been found to possess a number of interesting properties, such as the inhibition of excessive mammalian collagenolytic activity both *in vitro* and *in vivo.* See, for example, Golub et al. (1991).

It has been established that tetracyclines, which are rapidly absorbed and have a prolonged plasma half-life, exert biological effects independent of their antibacterial activity (Golub et al. 1991, Golub et al. 1992, Uitto et al. 1994). Such effects include inhibition of some but hot all animal and human derived matrix metalloproteinases.

Studies have suggested that, in some systems, certain tetracyclines and inhibitors of metalloproteinases can inhibit tumor progression (DeClerck et al. 1994) or angiogenesis (WIPO publication WO 92/12717; Maragoudakis et al. 1994). Zucker et al. (1985) showed that minocycline can inhibit melanoma cell activity in *vitro.* Some tetracyclines may exhibit cytostatic effects against some tumors (Kroon et al. 1984; van den Bogert et al. 1986). Pro-gelatinase A (MMP-2) has been reported to be associated with tumor spread (Yu et al. 1997). 6-demethyl-6-deoxy-4-de(dimethylamino)tetracycline (CMT-3) has been shown to suppress prostate and melanoma tumor growth and metastasis *in vivo* (Lokeshwar et al. 1998; Seftor et al. 1998).

Fungal growth is an important clinical problem, especially in patients whose immune system has been depressed. There are relatively few anti-fungal drugs presently available for clinical use. Additionally, fungi are developing resistance to the few anti-fungal agents approved for clinical use.

Currently available anti-fungal agents are generally quite toxic and can cause side effects such as renal impairment, phlebitis, and gastrointestinal disturbance. Some anti-fungal agents, such as Nystatin^{TM} are poorly absorbed by the gastrointestinal tract. Thus, there is a need for new and improved anti-fungal agents.

### SUMMARY OF THE INVENTION

It has now been discovered that these and other objectives can be achieved by the present invention, which provides a use of a chemically modified tetracycline (CMT), in an amount that is effective to inhibit the growth of fungus but that has substantially no antibacterial activity for the preparation of a medicament for inhibiting growth of a fungus.

### DETAILED DESCRIPTION OF INVENTION

The present invention provides compounds for use in a method of inhibiting the growth of fungus. The method includes the administration of a tetracycline compound that is effective in inhibiting the growth of fungus, but has substantially no antibacterial activity.

Fungus as defined herein includes any eukaryotic single celled organism characterized by the absence of chlorophyll and by the presence of a rigid cell wall. Preferred fungi are clinically significant fungi, meaning such fungi grow in or on mammals. Examples of clinically significant fungi include *Cryptococcus* species, *Candida albicans, Rhizopus* species, *Aspergillus fumigatus, Penicillium* species, *Absidia* species, *Scedosporium apiospermum, Phialophora verrucosa, Cunninghamella* species, *Tricothecium* species, *Ulocladium* species, and *Fonsecae* species.

Tetracycline compounds utilized in the method can inhibit the growth of more than one type of fungus. In addition, some tetracycline compounds inhibit the growth of some species of fungi more effectively than others. Accordingly, a single tetracycline compound or combination of tetracycline compounds can be administered to inhibit the growth of a plurality of fungi.

The tetracycline compound administered can be a non-antibacterial compound or a combination thereof. The tetracycline compound has the general structure indicated above.

In a preferred embodiment, a non-antibacterial tetracycline is administered. -Non-antibacterial tetracycline compounds are structurally related to the antibacterial tetracyclines, but have had their antibiotic activity substantially or completely eliminated by chemical modification. Chemically modified tetracyclines are referred to herein as CMTs.

For example, non-antibacterial tetracycline compounds are capable of achieving antibacterial activity comparable to that of tetracycline at concentrations at least about ten times, preferably at least about twenty five times, greater than that of tetracycline.

It has been discovered that tetracyclines having substantially no antibacterial activity can exhibit anti-fungal activity. Antibiotics, such as tetracyclines, are known to inhibit the growth of bacteria. However, it was generally believed that antibiotics, such as tetracyclines, in therapeutic doses, were unable to destroy nucleated (eukaryotic) cells such as fungus. It has now been discovered that tetracyclines having little or no antibacterial activity, such as CMTs, can exhibit excellent anti-fungal acitivity, while demonstrating low cytotoxicity.

Examples of chemically modified non-antibacterial tetracyclines (CMTs) include those that lack the dimethylamino group at position 4 of the tetracycline ring structure, e.g.,:
4-de(dimethylamino)tetracycline (CMT-1),
6-demethyl-6-deoxy-4-de(dimethylamino)tetracycline (CMT-3),
7-chloro-4-de(dimethylamino)tetracycline (CMT-4),
4-hydroxy-4-de(dimethylamino)tetracycline (CMT-6),
4-de(dimethylamino)-12α-deoxytetracycline (CMT-7),
6-deoxy-5α-hydroxy-4-de(dimethylamino)tetracycline (CMT-8),
4-dedimethylamino-12α-deoxyanhydrotetracycline (CMT-9),
7-dimethylamino-6-demethyl-6-deoxy-4-de(dimethylamino)tetracycline (CMT-10),
4-de(dimethylamino)-5-oxytetracycline,
5α,6-anhydro-4-hydroxy-4-de(dimethylamino)tetracycline,
4-de(dimethylamino)-11-hydroxy-12α-deoxytetracycline,
12α-deoxy-4-deoxy-4-de(dimethylamino)tetracycline, and
12α,4α-anhydro-4-de(dimethylamino)tetracycline.

Further examples of tetracyclines modified for reduced antibacterial activity include 6-α-benzylthiomethylenetetracycline, the mono-N-alkylated amide of tetracycline, 6-fluoro-6-demethyltetracycline, 11α-chlorotetracycline, tetracyclinonitrile (CMT-2), and tetracycline pyrazole (CMT-5).

The preferred chemically-modified tetracyclines include CMT-3, CMT-4, CMT-7 and CMT-8. CMT-3, CMT-7, and CMT-8 are most preferred.

Derivatives of CMTs as mentioned above can also be used. Combinations of tetracycline compounds, such as CMTs and/or their derivatives, can be used in the method of the invention. CMTs in combination with other tetracyclines and/or pharmaceutical compounds can also be employed.

For example, derivatives of CMT-3 include:

| | |
|---|---|
| CMT-308 | 9-AMINO |
| CMT-315 | 2-CONHCH₂-PIPERAZIN-1-YL |

Preferred derivatives are CMT-308 and CMT-315.

The tetracycline compounds administered can be in the form of pharmaceutically acceptable salts. The term "pharmaceutically acceptable salt" refers to a salt prepared from tetracycline compounds and pharmaceutically acceptable nontoxic acids or bases. The acids may be inorganic or organic acids of tetracycline compounds. Examples of inorganic acids include hydrochloric, hydrobromic, hydroiodic, sulfinic, and phosphoric acids. Examples of organic acids include carboxylic and sulfonic acids. The radical of the organic acids may be aliphatic or aromatic. Some examples of organic acids include formic, acetic, phenylacetic, propionic, succinic, glycolic, glucuronic, malcic, furoic, glutamic, benzoic, anthranilic, salicylic, phenylacetic, mandelic, embonic (pamoic), methanesulfonic, ethanesulfonic, panthenoic, benzenesulfonic, stearic, sulfanilic, alginic, tartaric, citric, gluconic, gulonic, arylsulfonic, and galactunonic acids. Appropriate organic bases may be selected, for example, from NN-dibenzylethylenediamine, chloroprocaine, choline, diethanolamine, ethylenediamine, meglumine (N-methylglucamine), and procaine. Suitable inorganic bases include sodium hydroxide and potassium hydroxide.

The inhibition of fungal growth occurs over a wide range of concentrations. The elective amount of tetracycline used according to the invention is an amount that is effectively inhibitory of fungal growth activity. An amount of tetracycline is effectively inhibitory to fungal growth activity if it significantly reduces fungal growth activity or fungal growth. Inhibition of fungal growth can also include the inhibition of the invasiveness of the fungus, for example, in mammalian tissue.

The minimal amount of tetracycline administered is the lowest amount capable of inhibiting or eliminating the growth of fungus. CMTs exhibit their anti-fungal inhibitory properties at concentrations that lead to relatively few side effects, and in some cases are substantially free of side effects. In general, CMTs can be used at higher levels than antibacterial tetracyclines, while avoiding the disadvantages associated with antibacterial activity, such as the indiscriminate killing of beneficial bacteria, and the emergence of resistant bacteria which often accompanies the use of antibacterial compounds over prolonged periods of time.

Preferably, the CMT has a low phototoxicity, or is administered in an amount that results in a serum level at which the phototoxicity is acceptable. Phototoxicity is a chemically-induced photosensitivity. Such photosensitivity renders skin susceptible to damage, e.g. sunburn, blisters, accelerated aging, erythemas and eczematoid lesions, upon exposure to light, in particular ultraviolet light.

Phototoxicity can be evaluated in terms of a photoirritancy factor (PIF), as described in the examples. A PIF value of about 1.0 indicates that a compound is considered to have no measurable phototoxicity.

The low phototoxic derivatives preferably have PIF values no greater than about 5, preferably no greater than about 2, more preferably no greater than about 1.5, most preferably no greater than about 1.2, and optimally about 1. An example of a CMT having a low phototoxicity is CMT-8.

The CMTs useful according to the invention possess a desirable but unusual combination of physicochemical properties, including activity, bioavailability, solubility, and reduction of side effects. These properties render the compounds particularly desirable for the inhibition of fungal growth activity in mammals. In addition, it is believed that the properties of hydrophilicity and hydrophobicity are well balanced in these compounds, enhancing their utility both *in vitro* and especially *in vivo,* while other compounds lacking such balance are of substantially less utility. Specifically, the compounds have an appropriate degree of solubility in aqueous media to permit absorption and bioavailability in the body, while also having a degree of solubility in lipids to permit traversal of the cell membrane to a putative site of action. CMT-3, which is highly lipophilic, may be readily absorbed by tissues during topical administration. The compounds are maximally effective if they can be delivered to the site or region of the fungal growth activity.

In the treatment of certain localized conditions, the degree of hydrophilicity of the CMT can be of lesser importance. Such compounds as tetracyclinonitrile (CMT-2) and 4-hydroxy-4-de(dimethylamino)tetracycline (CMT-6), which have low solubility in aqueous systems, can be used in direct or topical treatment of fungal growth. Animal experiments, in which adult rats are orally gavaged with these two CMTs, have shown no detectable blood levels of these compounds, indicating a lack of systemic absorption and/or extraordinarily rapid excretion.

The method of the invention can be used wherever the growth of fungus is to be diminished or eliminated, *including in vitro, ex vivo,* or *in vivo. In vitro* systems typically include cultured samples. *Ex vivo* biological systems typically include cells or organ systems removed from a living animal. *In vivo* uses are limited to biological systems that are living, and such uses typically include therapeutic or pharmaceutical interventions. Thus, embodiments of the invention in which a tetracycline compound is administered to a mammal are representative of *in vivo* methods.

The method of the invention is useful to treat medical conditions characterized by fungal growth activity. In particular, the invention is useful in the treatment (e.g., palliation, amelioration) of medical conditions characterized by excessive or pathological levels of fungal growth activity. Such conditions are often caused by a depressed immune system. For example, AIDS patients or cancer patients undergoing chemotherapy often suffer from development of fungal growth in the mouth and other areas of the body.

Accordingly the treatment of fungal growth in a living organism in need thereof is specifically provided. The method includes administration of CMTs to the living organism in an amount that is effective in inhibiting the growth of fungus, but has substantially no antibacterial activity.

In a preferred embodiment, the tetracycline is a CMT. Preferred CMTs include CMT-3, CMT-4, CMT-7, CMT-8, CMT-306, CMT-308, and CMT-315. CMT-3, CMT-7, CMT-8, CMT-308, and CMT-315 are most preferred. Administration can be topical or systemic. Effective amounts can be readily determined by those skilled in the art.

Mammals include, for example, humans, baboons and other primates, as well as pet animals such as dogs and cats, laboratory animals such as rats and mice, and farm animals such as horses, sheep, and cows. Humans are preferred.

Screening for antifungal CMTs with SABHI Agar culture showed that CMT-315, CMT-3, CMT-7 and CMT-308 are highly effective in inhibiting the growth of various tested fungi, including *Penicillium* species; *Aspergillus fumigatus*, *Rhizopus* species, and *Candida albicans* (Table 1 and 2). For example, CMT-315 significantly inhibited the growth of the four fungi including *Rhizopus sp.,* which is the fastest growing fungus in culture. The significance of this finding is that all these four tested fungi can cause human diseases.

For example, *Penicillium* species are responsible for pulmonary infection, skin infection, external otomycosis, mycotic keratitis, endocarditis, and cutaneous ulceration; *Aspergillus fumigatus* can cause allergic bronchopulmonary infection, fungus ball, invasive pulmonary infection, skin and nail infection, osteomyelitis, external otomycosis, mycotic keratitis, sinusitis, myocarditis, renal infection and brain abscess; *Rhizopus* species cause Rhinocerebral infection, pulmonary infection, mycotic keratitis, intraocular infeciton, orbital cellulitis, deep wound infection, external otomycosis, dermatitis, etc.

These fungi usually exist in air, soil, food, surfaces such as furniture, and sometimes in human body, such as in the mouth. The fungi enter tissues of living organisms and cause infection, especially in mammals when the immune system becomes weaker, such as in patients having diabetes, AIDS, cancers, transplants, etc.

The invention can also be practiced by including with the tetracycline compound one or more other therapeutic agents, such as any conventional anti-fungal agent. The combination of the tetracycline compound with such other agents can potentiate the therapeutic protocol. Numerous therapeutic protocols will present themselves in the mind of the skilled practitioner as being capable of incorporation into the method of the invention.

The preferred pharmaceutical composition for use in the method of the invention includes a combination of the tetracycline compound in a suitable pharmaceutical carrier (vehicle) or excipient as understood by practitioners in the art. Examples of carriers and excipients include starch, milk, sugar, certain types of clay, gelatin, stearic acid or salts thereof, magnesium or calcium stearate, talc, vegetable fats or oils, gums and glycols.

The tetracycline compound may be administered to mammals by sustained. release, as is known in the art. Sustained release administration is a method of drug delivery to achieve a certain level of the drug over a particular period of time. The level typically is measured by serum concentration.

Systemic administration can be enteral or parenteral. Enteral administration is a preferred route of delivery of the tetracycline, and compositions including the tetracycline compound with appropriate diluents, carriers, and the like are readily formulated. Liquid or solid (e.g., tablets, gelatin capsules) formulations can be employed.

Parenteral use (e.g., intravenous, intramuscular, subcutaneous injection) is also contemplated, and formulations using conventional diluents, carriers, etc., such as are known in the art can be employed to deliver the compound.

Alternatively, delivery of the tetracycline compound can include topical application. Compositions deemed to be suited for such topical use include as gels, salves, lotions, ointments and the like. In a preferred embodiment, the tetracycline compound is included in a mouthwash.

The maximal dosage for a mammal is the highest dosage that does not cause undesirable or intolerable side effects. Such doses can be readily determined by those skilled in the art. For example, CMTs can be systemically administered in a mammal in an amount of from about 0.1 mg/kg/day to about 60mg/kg/day, and preferably from about 1mg/kg/day to about 18mg/kg/day. The practitioner is guided by skill and knowledge in the field, and the present invention includes, without limitation, dosages that are effective to achieve the desired anti-fungal activity.

The appropriate dose of the tetracycline compound for topical administration can elso be readily determined by those skilled in the art. For example, topical administration of CMTs in amounts of up to about 25% (w/w) in a vehicle can be administered without any toxicity in a human. Amounts from about 0.1% to about 10% are preferred.

Most anti-fungal drugs presently available in clinics are much more toxic to mammals, such as humans, than antibacterial drugs. For example, amphotericin B may not only cause disorders of the digestion system, but also cause damage to the nervous system.

CMTs are usually less toxic to mammals than conventional anti-fungal compounds. For example, CMT-3 is being tested in cancer patients without evidence of side effects, except at unusually high doses. Also, CMT's are often absorbed into the system better than conventional anti-fungal drugs. Particular CMTs have only limited biodistribution, e.g. CMT-5. In such cases, topical administration is the preferred route of administration of the compound.

The following examples are provided to assist in a further understanding of the invention. The particular materials and conditions employed are intended to be further illustrative of the invention and are not limiting upon the reasonable scope thereof.

### EXAMPLE 1

The following example demonstrates a response of selected fungi to CMT-3, 4, 7, 8, and the following derivivatives of CMT-3: 302,303,306,308,309 and 315.

The following fungi were inoculated onto potato dextrose agar (PDA) from stock cultures and incubated aerobically at 30°C: *Aspergillus fumigatus* ATCC 1022, *Penicillium sp.* (laboratory isolate), *Candida albicans,* ATCC 14053, and *Rhizopus sp.*

A sterile cotton tipped applicator was moistened with sterile 0.9% saline and rolled over the surface of PDA slants of *Aspergillus fumigatus*, *Rhizopus sp.* and *Penicillium sp.* which demonstrated copious conidiogenesis. The conidia were suspended in 0.9% saline and the turbidity was adjusted to match a 0.5 MacFarland standard (equivalent to approximately 1.5 x 10⁸ cells). *Candida albicans* was suspended in saline and adjusted to 0.5 MacFarland in a similar manner. These suspensions were diluted 1:100 in sterile 0.9% saline.

SABHI Agar (Difco) pH 7.0 was prepared in 100ml amounts and sterilized at 121°C for 15 min. After the SABHI agar base cooled to 50°, 10 ml of each of the CMT substances were prepared in 10% DMSO at a concentration of 250 µg/ml. The CMT substances were than added at a final concentration of 25 µg/ml of agar base.

SABHI Agar plates of each CMT and SAHBI agar without CMT using dimethylsulphoxide (DMSO) as a control were inoculated with 10µl of conidia suspension of *Aspergillus fumigatus, Penicillium sp.* and *Rhizopus sp.* and 10µl suspension *of Candida albicans* prepared as described above. The plates were then incubated aerobically for 24 hour and for 48 hours at 30°C.

The results are set forth in Table 1 (24hr. incubation) and Table 2 (48 hr. incubation). The score table used for Tables 1 and 2 is set forth in Table 3.

**Table 1**

| Growth at 25 µg/ml compared to control at 24 hrs incubation | | | | | | | | | | | |
|---|---|---|---|---|---|---|---|---|---|---|---|
| Organism | 3 | 4 | 7 | 8 | 302 | 303 | 306 | 308 | 309 | 315 | DMSO |
| Aspergillus Fumigatus | 0 | 0 | 0 | ± | 0 | ± | 1 | 0 | ± | 0 | 3 |
| Penicillium Sp. | 0 | 3 | 3 | 3 | 3 | 3 | 3 | 0 | 3 | 0 | 4 |
| Rhizopus sp. | 3 | 4 | 4 | 4 | 4 | 4 | 4 | 4 | 4 | 1 | 4 |
| Candida Albicans | 1 | 1 | 0 | 4 | 4 | 3 | 3 | 4 | 4 | 0 | 4 |

**Table 2**

| Growth at 25µg/ml compared to control at 48 hours | | | | | | | | | | | |
|---|---|---|---|---|---|---|---|---|---|---|---|
| Organism | 3 | 4 | 7 | 8 | 302 | 303 | 306 | 308 | 309 | 315 | DMSO |
| Aspergillus Fumigatus | 4 | 1 | 4 | 4 | 4 | 4 | 4 | 1 | 4 | 0 | 4 |
| Penicillium Sp. | 4 | 0 | 4 | 4 | 4 | 4 | 4 | 0 | 4 | 0 | 4 |
| Rhizopus sp. | 1 | 4 | 3 | 4 | 4 | 4 | 4 | 4 | 4 | 1 | 4 |
| Candida Albicans | 4 | 4 | 0 | 4 | 4 | 4 | 4 | 4 | 4 | 0 | 4 |

**Table 3**

| Inhibition Score and Grading of Fungal Growth | | |
|---|---|---|
| Growth Grade | Inhibition Score | Description |
| 4 | 0% | Level of growth in the absence of anti-fungal agent (control). |
| 3 | 25% | 25% reduction in growth of colonies compared to control. |
| 2 | 50% | 50% reduction in growth of colonies compared to control. |
| 1 | 75% | 75% reduction in growth in colonies compared to control. |
| 0 | 100% | complete inhibition of growth. |

CMT-315 yielded the best results with activity against all the fungi tested. CMT-308 demonstrated activity against *Aspergillus fumigatus* and *Penicillium sp..* CMT-4 demonstrated activity against *Penicillium sp.,* and *Aspergillus f.* CMT-7 demonstrated strong activity against *Candida albicans.* CMT-3 inhibited *Rhizopus sp.,* which is the most rapidly growing of the fungi, and can cause Rhinocerebral infection, pulmonary infection, mycotic keratitis, intraocular infection, orbital cellulitis, deep wound infection, external otomycosis, dermatitis, etc.

### EXAMPLE 2

This example demonstrates a direct comparison between CMT-3 and Amphotericin B (AmB), a conventional anti-fungal agent, in the inhibition of *Aspergillus f.* The plates were prepared as described above, using 0.125, 0.5, 0.50, 1.00 and 2.00 concentrations of each of the drugs tested. DMSO was used as a control

The results are shown in Table 4 below. The results were graded according to the criteria set forth in Table 3.

**Table 4**

| Conc. (µg/ml) | 0.125 | 0.25 | 0.50 | 1.00 | 2.00 |
|---|---|---|---|---|---|
| CMT-3 | 4 | 2 | 1 | ±0 | 0 |
| AmB | 4 | 2 | 1 | 0 | 0 |

The results demonstrate that at various concentrations, the CMT-3 inhibited growth of *Aspergillus f.* as effective as AmB. At a concentration of 1.0 µg/ml, AmB inhibited 100% of fungal growth, while CMT-3 inhibited 95% of growth. At 2.0 µg/ml, both AmB and CMT-3 inhibited 100% of growth. Importantly, unlike AmB, CMT-3 demonstrates very little toxicity in vivo at 2.0 µg/ml concentration.

### EXAMPLE 3

This example demonstrates the concentration of anti-fungal agent required to reduce the growth of the fungus by 50% in vitro (IC50) and the minimum concentration required to completely inhibit the growth of the fungus in vitro (MIC). CMTs utilized in the method of the invention, i.e CMT-3 and CMT-8 were compared to Doxycycline and Amphotericin B on microplate agar gels.

Each drug was dissolved in DMSO (1.0 mg/ml) as a stock solution and stored at -20°C. Just prior to use, each stock solution was thawed and diluted in DMSO to produce 6 different 100x concentrations. Potato dextrose agar was dissolved in distilled water (39 g/L) and sterilized at 138°C (250°F) for 15 min. The agar solution was mixed with each drug (in a water bath at 60°C) to make a series of final concentrations, i.e. 0.00, 0.25, 0.50, 1.00, 2.00, 4.00 µg/ml. The mixtures were then transferred to 24-well plates (1 ml/well). After the gel had formed, the fungus in PBS (spore count = 1-5x10⁴/ml) was inoculated by pipetting 10µl onto each gel. The plates were incubated at 30°C for different times, depending on the requirement of each species, e.g. 24 hours for *Penicillium, Rhizopus, Tricothecium, Ulocladium, Absidia, Aspergilus, Candida, Cunninghamella,* 3 days for *Scedosporium,* and 5 days for *Fonsecae* and *Phialophora.*

The MICs and IC50s for the 11 different fungi are set forth in Table 5. "*" indicates better than or similar results to Amphotericin B. "NI" indicates no detectable inhibition.

**Table 5**

| | **IC50(µg/ml)** | **MIC(µg/ml)** |
|---|---|---|
| **Candida Albicans** | | |
| AmB | 0.5 | 1.0 |
| CMT-3 | 1.0 | 2.0 |
| CMT-8 | NI | NI |
| Doxy | NI | NI |
| | | |

| **Rhizopus Species** | | |
|---|---|---|
| AmB | 0.4 | 1.0 |
| CMT-3 | 0.8 | 2.0 |
| CMT-8 | NI | NI |
| Doxy | NI | NI |
| | | |

| **Aspergilus Fumigatus** | | |
|---|---|---|
| AmB | 0.8 | 2.0 |
| CMT-3 | 0.5 | 1.0* |
| CMT-8 | NI | NI |
| Doxy | NI | NI |
| | | |

| **Penicillium Species** | | |
|---|---|---|
| AmB | 0.12 | 0.25 |
| CMT-3 | 0.2 | 0.5 |
| CMT-8 | 2.0 | >4 |
| Doxy | | NI |
| | | |

| **Absidia Species** | | |
|---|---|---|
| AmB | 1.0 | 4.0 |
| CMT-3 | 1.5 | 4.0* |
| CMT-8 | NI | NI |
| Doxy | NI | NI |
| | | |

| **Scedosporium Apiospermum** | | |
|---|---|---|
| AmB | 4.0 | >4 |
| CMT-3 | 0.2 | 1.5* |
| CMT-8 | 2.0 | >4 |
| Doxy | NI | NI |
| | | |

| **Phialophora Verrucosa** | | |
|---|---|---|
| AmB | NI | NI |
| CMT-3 | 1.5 | 4.0* |
| CMT-8 | NI | NI |
| Doxy | NI | NI |
| | | |

| **Cunninghamella Species** | | |
|---|---|---|
| AmB | NI | NI |
| CMT-3 | 2.0 | 4.0* |
| CMT-8 | NI | NI |
| Doxy | NI | NI |
| | | |

| **Tricothecium Species** | | |
|---|---|---|
| AmB | NI | NI |
| CMT-3 | 0.2 | 1.5* |
| CMT-8 | 0.7 | 2.0 |
| Doxy | 4.0 | >4 |

| **Ulocladium Species** | | |
|---|---|---|
| AmB | 1.0 | 2.0 |
| CMT-3 | 0.25 | 1.0* |
| CMT-8 | 2.0 | >4 |
| Doxy | NI | NI |
| | | |

| **Fonsecae Species** | | |
|---|---|---|
| AmB | 4.0 | >4.0 |
| CMT-3 | 1.0 | 4.0* |
| CMT-8 | NI | NI |
| Doxy | NI | NI |

Thus, CMT-3 was effective on all 11 tested fungi, and CMT-8 had effects on some of these fungi. However, for 8 fungi out of the 11 different species of fungi, Amphotericin B showed the same or less antifungal activity than CMT-3. Doxy had essentially no detectable antifungal activity in this experiment.

### EXAMPLE 4

This example demonstrates the antifungal activity of CMT-3 and Amphotericin B *in vitro* as being fungistatic (i.e. arresting the growth of the fungus) or fungicidal (i.e. killing the fungus).

In the pre-treatment phase of the experiment, *Penicillium* spores were suspended in PBS to achieve a spore count of 10⁷/ml. CMT-3 and Amphotericin B were dissolved in DMSO to reach a concentration of 1.0 mg/ml as stock solutions. 10 or 50 µl aliquots of these stock solutions were added to the incubation mixture (containing 1.0 ml of 10⁷/ml of *Penicillium* spores in PBS) to achieve a final concentration of 10 µg/ml or 50 µg/ml, respectively, for both drugs. The various incubations of *Penicillium* were carried out for 24 hours at 30°C.

After the pre-treatment phase, the reaction mixtures were diluted 1000 times with PBS, reducing the concentration of both drugs to 0.01 µg/ml or 0.05 µg/ml, and reducing the *Penicillium* spore count to 10⁴/ml. These drug concentrations of both CMT-3 and Amphotericin B would not be expected to inhibit the growth of the viable *Pencillium* spores.

Controls were then prepared. Before incubation, each tube was either not diluted further, or diluted to ½ or ¼ with PBS to produce tubes with three different spore counts, ie, 10⁴/ml, or 0.5 x 10⁴/ml, or 0.25 x 10⁴/ml. These cultures were then inoculated on potato dextrose agar gels in 24-well plates, and incubated at 30°C for 48 hours to determine the rate of growth of the fungus as described before.

The controls were prepared from the suspension in the pre-treatment phase containing only *Penicillium* spores 10⁷/ml, and PBS. This control was diluted by 1000 times with PBS to produce a spore count of 10⁴/ml. 1.0 ml of this diluted spore suspension was added to eight tubes. The stock solutions of CMT-3 and Amphotericin B, and DMSO were also diluted by 1000 times with PBS (the new concentration being 1.0 µg/ml for both drugs and 0.1% for DMSO), and 10 or 50 µl of these solutions was added into the above tubes. The final concentrations in each tube was either 0.01 µg/ml or 0.05 µg/ml for both drugs (CMT-3 or AmB), or 0.001 % or 0.005% for DMSO. These tubes were further treated as described above to determine the growth of the fungus as controls.

The results demonstrated that all controls, including the concentration of 0.01 and 0.05 µl/ml of both drugs (CMT-3 and Amphotericin B), showed the same growth rate of *Penicillium* as the cultures without drugs, demonstrating that these low concentrations of both drugs did not inhibit the growth of the fungus in these control cultures.

Cultures of the *Penicillium*, after pretreatment with 10 and 50 µl/ml of Amphotericin B, showed the same rate of growth as PBS and DMSO controls during the subsequent incubation phase of the experiment, indicating that this drug did not kill the spores during the pre-treatment phase.

In contrast, cultures of *Penicillium* after pretreatment with 10 and 50 µl/ml of CMT-3, showed little or no growth on the agar gels compared with the controls, demonstrating that CMT-3 did kill the fungal spores during the pre-treatment phase.

Thus, Amphotericin B exhibited fungistatic activity, i.e. fungal growth was arrested but the fungal spores were not killed. On the other hand, CMT-3 exhibited fungicidal activity against *Penicillium,* killing the fungus.

### References

DeClerck YA, Shimada H, Taylor SM, and Langley KE, "Matrix metalloproteinases and their inhibitors in tumor progression," *Annals NY Acad Sci* 732:222-232 (1994).

Golub LM, Ramamurthy NS, McNamara TF, Greenwald RA, and Rifkin BR, "Tetracyclines inhibit connective tissue breakdown: New therapeutic implications for an old family of drugs," *Crit Rev Oral Biol Med* 2(2):297-322 (1991).

Golub LM, Sorsa T, and Suomalainen K, *Curr Opin Dent* 2:80-90 (1992).

Kroon AM, Dontje BHJ, Holtrop M, and van den Bogert C, "The mitochondrial genetic system as a target for chemotherapy: tetracyclines as cytostatics," *Cancer Letts* 25(1):33-40 (1984).

Lokeshwar BL, Selzer MG, Dudak SM, Block NL, and Golub LM, "Inhibition of tumor growth and metastasis by oral administration of a non-antimicrobial tetracycline analog (CMT-3) and doxycycline in a metastatic prostate cancer model," *Cancer Res* (1998).

Maragoudakis ME, Peristeris P, Missirlis E, Aletras A, Andriopoulou P, and Haralabopoulos G, *Annals NY Acad Sci* 732:280-293 (1994).

Mitscher LA, *The Chemistry of the Tetracycline Antibiotics,* Ch. 6, Marcel Dekker, New York (1978).

Seftor REB, Seftor EA, DeLarco JE, Kleiner DE, Leferson J, Stetler-Stevenson WG, McNamara TF, Golub LM, and Hendrix MJC, "Chemically-modified tetracyclines inhibit human melanoma cell invasion and metastasis," *Clin Exp Metastasis* 16 (1998).

Uitto VJ, Firth JD, Nip L, and Golub LM, *Annals NY Acad Sci* 732:140-151 (1994).

van den Bogert C, Dontje BHJ, Holtrop M, Melis TE, Romijn JC, van Dongen JW, and Kroon AM, "Arrest of the proliferation of renal and prostate carcinomas of human origin by inhibition of mitochondrial protein synthesis," *Cancer Res* 46(7):3283-3289 (1986).

Yu AE, Hewitt RE, Connor EW, Stetler-Stevenson WG, "Matrix metalloproteinases, Novel targets for directed cancer therapy," *Clin Pharmacol* 11:229-244 (1997).

Zucker S, Lysick RM, Ramamurthy NS, Golub LM, Wieman JM, and Wilkie DP, "Diversity of plasma membrane proteinases in mouse melanoma cells: Inhibition of collagenolytic activity and cytolytic activity by minocycline," *J Natl Cancer Inst* 75:517-525 (1985).

## Claims

1. Use of a chemically-modified tetracycline compound (CMT) selected from the group consisting of CMT-3, CMT-4, CMT-7, CMT-8, CMT-308, CMT-315 and combinations thereof for the preparation of a medicament for inhibiting growth of a fungus in a mammal in need thereof.

2. Use according to claim 1 wherein said fungus is selected from the group consisting of *Cryptococcus* species, *Candida albicans, Rhizopus* species, *Aspergillus fumigatus, Penicillium* species, *Absidia* species, *Scedosporium apiospermum, Phialophora verrucosa, Cunninghamella* species, *Tricothecium* species, *Ulocladium* species, *Fonsecae* species, and combinations thereof.

3. Use according to claim 1 wherein said CMT is CMT-315.

4. Use according to claim 1 wherein said fungus is selected from the group consisting of *Rhizopus* species, *Absidia* species, *Scedosporium apiospermum, Phialophora verrucosa, Cunninghamella* species, *Tricothecium* species*, Ulocladium* species, *Fonsecae* species, or a combination thereof, and wherein said CMT is CMT-3.

5. Use according to claim 1 wherein said fungus is *Aspergillus fumigatus, Penicillium* species, *Rhizopus* species, *Candida albicans,* or a combination thereof, and wherein said CMT is CMT-315.

6. Use according to claim 1, wherein said fungus is *Penicillium* species and said CMT is CMT-4.

7. Use according to claim 1 wherein said fungus is *Candida albicans* and said CMT is CMT-7.

8. Use according to claim 1 wherein said fungus is *Aspergillus fumigatus, Penicillium* species or a combination thereof, and said CMT is CMT-308.

9. Use according to claim 1 wherein said fungus is *Candida albicans, Penicillium* species, *Scedosporium apiospermum, Tricothecium* species, *Ulocladium* species, or a combination thereof and said CMT is CMT-8.

10. Use according to claim 1, wherein said mammal is a human.

11. Use according to claim 1 wherein said medicament is to be administered systemically.

12. Use according to claim 11 wherein said CMT is to be administered in an amount from 0.1 mg/kg/day to 60 mg/kg/day.

13. Use according to Claim 11 wherein said CMT is to be administered in an amount from 1 mg/kg/day to 18 mg/kg/day.

14. Use according to claim 1 wherein said administration is topical.

15. Use according to claim 14 wherein said CMT is to be administered in a mouthwash.

## Patentansprüche

1. Verwendung einer chemisch modifizierten Tetracyclinverbindung (CMT), die aus der Gruppe ausgewählt ist, die aus CMT-3, CMT-4, CMT-7, CMT-8, CMT-308, CMT-315 und Kombinationen davon besteht, zur Herstellung eines Medikaments zur Hemmung des Wachstums eines Pilzes bei einem Säuger, der dieses Medikaments bedarf.

2. Verwendung gemäß Anspruch 1, wobei der Pilz aus der Gruppe ausgewählt ist, die aus *Cryptococcus* species, *Candida albicans, Rhizopus* species, *Aspergillus fumigatus, Penicillium* species, *Absidia* species, *Scedosporium apiospermum, Phialophora verrucosa, Cunninghamella* species, *Tricothecium* species, *Ulocladium* species, *Fonsecae* species und Kombinationen davon besteht.

3. Verwendung gemäß Anspruch 1, wobei es sich bei dem CMT um CMT-315 handelt.

4. Verwendung gemäß Anspruch 1, wobei der Pilz aus der Gruppe ausgewählt ist, die aus *Rhizopus* species, *Absidia* species, *Scedosporium apiospermum, Phialophora verrucosa, Cunninghamella* species, *Tricothecium* species, *Ulocladium* species, *Fonsecae* species oder einer Kombination davon besteht und es sich bei dem CMT um CMT-3 handelt.

5. Verwendung gemäß Anspruch 1, wobei es sich bei dem Pilz um *Aspergillus fumigatus, Penicillium* species, *Rhizopus* species, *Candida albicans* oder eine Kombination davon und bei dem CMT um CMT-315 handelt.

6. Verwendung gemäß Anspruch 1, wobei es sich bei dem Pilz um *Penicillium* species und bei dem CMT um CMT-4 handelt.

7. Verwendung gemäß Anspruch 1, wobei es sich bei dem Pilz um *Candida albicans* und bei dem CMT um CMT-7 handelt.

8. Verwendung gemäß Anspruch 1, wobei es sich bei dem Pilz um *Aspergillus fumigatus, Penicillium* species oder eine Kombination davon und bei dem CMT um CMT-308 handelt.

9. Verwendung gemäß Anspruch 1, wobei es sich bei dem Pilz um *Candida albicans, Penicillium* species, *Scedosporium apiospermum, Tricothecium* species, *Ulocladium* species oder eine Kombination davon und bei dem CMT um CMT-8 handelt.

10. Verwendung gemäß Anspruch 1, wobei der Säuger ein Mensch ist.

11. Verwendung gemäß Anspruch 1, wobei das Medikament systemisch verabreicht werden soll.

12. Verwendung gemäß Anspruch 11, wobei das CMT in einer Menge von 0,1 mg/kg/Tag bis 60 mg/kg/Tag verabreicht werden soll.

13. Verwendung gemäß Anspruch 11, wobei das CMT in einer Menge von 1 mg/kg/Tag bis 18 mg/kg/Tag verabreicht werden soll.

14. Verwendung gemäß Anspruch 1, wobei die Verabreichung topisch erfolgt.

15. Verwendung gemäß Anspruch 14, wobei das CMT in einem Mundwasser verabreicht werden soll.

## Revendications

1. Utilisation d'un composé de tétracycline chimiquement modifié (CMT) choisi dans le groupe consistant en CMT-3, CMT-4, CMT-7, CMT-8, CMT-308, CMT-315 et leurs combinaisons pour la préparation d'un médicament pour inhiber la croissance d'un champignon dans un mammifère qui en a besoin.

2. Utilisation selon la revendication 1 où ledit champignon est choisi dans le groupe consistant en les espèces de *Cryptococcus, Candida albicans,* les espèces de *Rhizopus, Aspergillus fumigatus,* les espèces de *Penicillium,* les espèces de *Absidia, Scedosporium apiospermum, Phialophora verrucosa,* les espèces de *Cunninghamella,* les espèces de *Tricothecium,* les espèces de *Ulocladium,* les espèces de *Fonsecae,* et leurs combinaisons.

3. Utilisation selon la revendication 1 où ledit CMT est CMT-315.

4. Utilisation selon la revendication 1 où ledit champignon est choisi dans le groupe consistant en les espèces de *Rhizopus,* les espèces de *Absidia, Scedosporium apiospermum, Phialophora verrucosa,* les espèces de *Cunninghamella,* les espèces de *Tricothecium,* les espèces de *Ulocladium,* les espèces de *Fonsecae,* ou une combinaison de ceux-ci, et où ledit CMT est CMT-3.

5. Utilisation selon la revendication 1 où ledit champignon est *Aspergillus fumigatus,* une espèce de *Penicillium,* une espèce de *Rhizopus, Candida albicans,* ou une combinaison de ceux-ci, et où ledit CMT est CMT-315.

6. Utilisation selon la revendication 1 où ledit champignon est une espèce de *Penicillium* et ledit CMT est CMT-4.

7. Utilisation selon la revendication 1 où ledit champignon est *Candida albicans* et ledit CMT est CMT-7.

8. Utilisation selon la revendication 1 où ledit champignon est *Aspergillus fumigatus,* une espèce de *Penicillium,* ou une combinaison de ceux-ci, et ledit CMT est CMT-308.

9. Utilisation selon la revendication 1 où ledit champignon est *Candida albicans,* une espèce de *Penicillium*, *Scedosporium apiospermum*, une espèce de *Tricothecium,* une espèce de *Ulocladium,* ou une combinaison de ceux-ci, et ledit CMT est CMT-8.

10. Utilisation selon la revendication 1 où ledit mammifère est un humain.

11. Utilisation selon la revendication 1 où ledit médicament est destiné à être administré par voie systémique.

12. Utilisation selon la revendication 11 où ledit CMT est destiné à être administré en une quantité de 0,1 mg/kg/jour à 60 mg/kg/jour.

13. Utilisation selon la revendication 11 où ledit CMT est destiné à être administré en une quantité de 1 mg/kg/jour à 18 mg/kg/jour.

14. Utilisation selon la revendication 1 où ladite administration est topique.

15. Utilisation selon la revendication 14 où ledit CMT est destiné à être administré dans un bain de bouche.
